Europäisches Patentamt

European Patent Office

Office européen des brevets

(19)

(11)   EP 0 950 050 B1

(12)   FASCICULE DE BREVET EUROPEEN

(45) Date de publication et mention
de la délivrance du brevet:
**26.10.2005   Bulletin 2005/43**

(51) Int Cl.$^7$: **C07D 211/70**, A61K 31/44

(21) Numéro de dépôt: **97952986.4**

(86) Numéro de dépôt international:
**PCT/FR1997/002393**

(22) Date de dépôt: **23.12.1997**

(87) Numéro de publication internationale:
**WO 1998/028271 (02.07.1998 Gazette 1998/26)**

(54) **PROCEDE POUR LA CRISTALLISATION D'UN DERIVE DE TETRAHYDROPYRIDINE ET FORMES CRISTALLINES AINSI OBTENUES**

VERFAHREN ZUR KRISTALLISIERUNG EINES TETRAHYDROPYRIDINDERIVATES UND DIE SO ERHALTENEN KRISTALLINEN FORMEN

METHOD FOR THE CRYSTALLISATION OF A TETRAHYDOPYRIDIN DERIVATIVE AND RESULTING CRYSTALLINE FORMS

(84) Etats contractants désignés:
**AT BE CH DE DK ES FI FR GB GR IE IT LI LU MC NL PT SE**
Etats d'extension désignés:
**LT LV SI**

(30) Priorité:  **23.12.1996  FR 9615904**

(43) Date de publication de la demande:
**20.10.1999   Bulletin 1999/42**

(73) Titulaire: **Sanofi-Aventis**
**75013 Paris (FR)**

(72) Inventeurs:
• **CARON, Antoine**
**F-34560 Montbazin (FR)**
• **FRANC, Bruno**
**F-30650 Saze (FR)**
• **MONNIER, Olivier**
**F-34560 Villeveyrac (FR)**

(74) Mandataire: **Gillard, Marie-Louise et al**
**Cabinet Beau de Loménie,**
**158, rue de l'Université**
**75007 Paris (FR)**

(56) Documents cités:
**EP-A- 0 101 381**

Remarques:
Le dossier contient des informations techniques présentées postérieurement au dépôt de la demande et ne figurant pas dans le présent fascicule.

**Description**

La présente invention concerne un procédé pour la cristallisation d'un dérivé de tétrahydropyridine, les nouvelles formes cristallines ainsi obtenues et une composition pharmaceutique contenant, en tant que principe actif, ledit dérivé de tétrahydropyridine sous une forme cristalline déterminée.

Plus particulièrement, la présente invention se rapporte à un procédé pour la cristallisation du chlorhydrate de la 1-[2-(2-naphtyl)éthyl]-4-(3-trifluorométhylphényl)-1,2,3,6-tétrahydropyridine, à trois formes cristallines de ce produit, à un mélange défini de deux de ces trois formes ainsi qu'à une composition pharmaceutique contenant une desdites formes ou un mélange de deux d'entre elles.

La 1-[2-(2-naphtyl)éthyl]-4-(3-trifluorométhylphényl)-1,2,3,6-tétrahydropyridine, ci-après désignée par son numéro de code SR 57746, et ses sels pharmaceutiquement acceptables ont été décrits pour la première fois dans EP 0 101 381 comme étant des agents anorexigènes et, par la suite, comme anti-anxiodépresseurs (US 5 026 716), anticonstipants (US 5 109 005), neurotrophiques (US 5 270 320), anti-radicaux libres (US 5 292 745) et cardioprotecteurs (US 5 378 709).

Dans EP 0 101 381, le SR 57746 est décrit sous forme de chlorhydrate, ci-après désigné SR 57746 A, et ce sel a été utilisé dans des essais précliniques et cliniques sur des volontaires sains. Le SR 57746 A, selon ce document, est isolé par cristallisation dans l'éthanol, notamment dans l'éthanol absolu.

Dans les essais précliniques, notamment dans les tests de pharmacologie animale et de toxicologie, le SR 57746 A a montré une activité et un comportement constants. De même, les études de pharmacocinétique chez les animaux ont donné des résultats constants et reproductibles.

Par contre, lors des études cliniques effectuées sur des volontaires sains (Phase I), il a été constaté que le SR 57746 A montre une grande variabilité dans les concentrations plasmatiques et dans les effets pharmacodynamiques du principe actif.

Dans les premiers essais cliniques chez des malades atteints de maladies très graves, notamment de sclérose latérale amyotrophique, la dose de SR 57746 A a été maintenue très basse, à savoir 2 mg/jour et à cette dose le produit s'est montré prometteur (W. G. Bradley, présentation intitulée "New drugs for amyotrophic lateral sclerosis", American Academy of Neurology meeting, March 23-30, 1996; pages 240-23/240-28).

Il a été par ailleurs constaté que dans la préparation de plus grandes quantités de SR 57746 A selon le procédé d'isolement décrit dans EP 0 101 381 on ne réussit pas à obtenir un produit avec des caractéristiques constantes permettant de pallier les inconvenients notés lors des études cliniques de Phase I.

Plus particulièrement, il a été constaté que selon le procédé d'isolement décrit dans EP 0 101 381 on obtient le SR 57746 A constitué par des cristaux dont la taille n'est pas constante et, notamment, est supérieure à 150 micromètres, plus particulièrement est de 150-600 micromètres pour au moins environ 75% des cristaux.

En outre, il a été constaté qu'en opérant selon la méthode décrite dans EP 0 101 381, on obtient un SR 57746 A constitué par au moins 3 différentes formes, comme il a été démontré par analyse calorimétrique différentielle.

Il a été enfin constaté que dans les différents lots de fabrication de SR 57746 A les rapports respectifs des différentes formes ne sont pas constants, ce qui rend difficile la maîtrise des caractéristiques de la matière première pour la fabrication de compositions pharmaceutiques.

Il a été maintenant trouvé qu'en effectuant la cristallisation du SR 57764 A dans des conditions convenables et constantes en termes de solvant, de vitesse d'agitation et de vitesse de refroidissement, il est possible d'isoler le composé sous trois formes cristallines différentes ou sous forme d'un mélange de deux de ces trois formes dans des rapports fixes et reproductibles.

Plus particulièrement, il a été trouvé que

- en refroidissant sans agitation une solution de SR 57746 A dans un mélange éthanol/acide chlorhydrique concentré, on obtient la Forme I du SR 57746 A:

- en refroidissant une solution de SR 57746 A dans l'éthanol absolu ou dans un mélange acétate d'éthyle/eau dans des conditions controlées de vitesse de refroidissement et de vitesse d'agitation, on obtient la Forme II du SR 57746 A;
- en refroidissant une solution de SR 57746 A dans du diméthylsulfoxyde, on obtient la Forme III du SR 57746 A;
- en refroidissant une solution de SR 57746 A dans un mélange éthanol/eau, on obtient un mélange de Forme 1 et de Forme III dans des proportions fixes et reproductibles.

Il a été également trouvé que ces nouvelles formes cristallines, seules ou en mélanges fixes de deux d'entre elles, sont absorbées de façon uniforme et reproductible et permettent l'établissement aisé du dosage optimal du principe actif. Au déla des améliorations au niveau pharmacocinétique et pharmacodynamique, le fait de pouvoir maîtriser la reproductibilité de la composition sous forme cristalline du SR 57746 A est très avantageux en vue de la commercialisation du médicament.

Il a été enfin trouvé que lorsque les nouvelles formes cristallines ou les mélanges de deux d'entre elles sont formés de cristaux de taille très petite, notamment si ils sont micronisés, l'activité du principe actif augmente sensiblement et son absorption est uniforme et constante, ce qui permet ainsi à la fois l'administration de faibles dosages avec une très bonne réponse thérapeutique et la maîtrise totale des effets secondaires potentiels.

Les figures en annexe montrent les thermogrammes obtenus en soumettant la Forme I, la Forme II, la Forme III et un mélange Forme I/Forme III dans un rapport 65,7/34,3 à une analyse calorimétrique différentielle.

La Figure 1 montre le thermogramme de la Forme I du SR 57746 A, préparée selon l'Exemple 1, obtenu par analyse calorimétrique différentielle de 50°C à 180°C. Ce thermogramme montre une température de transition solide-solide de 148-149°C.

La Figure 2 montre le thermogramme de la Forme II du SR 57746 A, préparée selon l'Exemple 2, obtenu par analyse calorimétrique différentielle de 50°C à 180°C. Ce thermogramme montre une température de transition solide-solide de 153-155°C.

La Figure 3 montre le thermogramme de la Forme III du SR 57746 A, préparée selon l'Exemple 3, obtenu par analyse calorimétrique différentielle de 50°C à 180°C. Ce thermogramme montre une température de transition solide-solide de 141-142°C.

La Figure 4 montre le thermogramme du mélange Forme I/Forme III, préparé selon l'Exemple 4, obtenu par analyse calorimétrique différentielle de 50°C à 180°C. Ce thermogramme montre les températures de transition solide-solide des deux formes.

Ainsi, selon un de ses aspects, la présente invention a pour objet un procédé pour la cristallisation du chlorhydrate de 1-[2-(2-naphtyl)éthyl]-4-(3-trifluorométhylphényl)-1,2,3,6-tétrahydropyridine caractérisé en ce que

(a) on dissout ledit composé par chauffage dans un solvant choisi parmi les alcanols de 1 à 3 atomes de carbone, les cétones de 3 à 6 atomes de carbone, le diméthylsulfoxyde ou l'acétate d'éthyle, ledit solvant contenant éventuellement de 5 à 30% en volume d'eau ou d'acide chlorhydrique aqueux;

(b) on refroidit la solution ainsi obtenue jusqu'à -10/+10°C à une vitesse de 3 à 100°C/heure, sous une agitation de 0 à 600 tr/minute; et

(c) on isole le produit ainsi obtenu et on le micronise.

Le procédé de la présente invention est conduit selon le mode opératoire classique des techniques de cristallisation, mais le type de solvant, la vitesse de refroidissement, l'absence ou la présence d'eau ainsi que la vitesse d'agitation constituent des paramètres essentiels pour l'obtention, de façon reproductible, d'une forme cristalline plutôt que d'une autre ou, toujours de façon reproductible, d'un mélange de deux formes dans des rapports fixes.

Dans l'étape (a), un chlorhydrate de 1-[2-(2-naphtyl)éthyl]-4-(3-trifluorométhylphényl)-1,2,3,6-tétrahydropyridine, par exemple le produit brut obtenu selon la méthode décrite dans EP 0 101 381, est chauffé, de préférence au reflux, dans le solvant choisi, éventuellement en présence d'eau.

La présence de l'eau peut se révéler utile pour solubiliser complètement le SR 57746 A. Ainsi, par exemple, dans le méthanol et dans l'éthanol le produit se solubilise complètement, à une concentration raisonnable (par exemple 15-150 g/l), alors que, aux mêmes concentrations, il ne passe pas complètement en solution dans l'acétone, la méthyléthylcétone, l'isopropanol et l'acétate d'éthyle. Dans ces solvants, il est alors suffisant d'ajouter 5 à 30% d'eau pour que la solubilisation complète ait lieu à la température de reflux. Le pourcentage d'eau ne doit cependant pas être trop élevé pour éviter un excès de solubilisation et une perte de produit final.

Selon un mode opératoire avantageux, le solvant utilisé est choisi parmi les mélanges (v/v) méthanol/eau de 100/0 à 70/30, éthanol/eau de 100/0 à 70/30, acétone/eau de 95/5 à 70/30, méthyléthylcétone/eau de 95/5 à 80/20, acétate d'éthyle/eau de 95/5 à 70/30 ou le diméthylsulfoxyde.

La concentration du SR 57746 A dans le solvant choisi depend, comme il a été mentionné ci-dessus, de la solubilité. Elle peut aller d'environ 15 - 100 g/l pour les mélanges acétate d'éthyle/eau à 150 - 300 g/l pour l'éthanol et pour les mélanges éthanol/eau.

Avantageusement, le SR 57746 A est dissous à une concentration de 5-150 g/l, de préférence de 100-150 g/l dans de l'éthanol ou dans un mélange éthanol/eau environ 90/10 ou dans le méthanol, ou bien à une concentration d'environ 60 g/l dans un mélange acétone/eau environ 90/10, de 100-125 g/l dans un mélange méthyléthylcétone/eau environ 95/15 ou d'environ 15 g/l dans un mélange acétate d'éthyle/eau environ 90/10. Dans ces conditions, la solubilisation dans le solvant au reflux est totale.

Dans l'étape (b), la solution ainsi obtenue est refroidie, éventuellement sous agitation, avec un contrôle de la vitesse de refroidissement et, s'il y a agitation, de la vitesse d'agitation car l'obtention d'une forme cristalline convenable dépend en grande partie de ces deux paramètres.

Lorsque la cristallisation est effectuée sous agitation, on utilise de préférence un mobile d'agitation à palette (ci-après aussi appelé "impeller") permettant un mouvement de rotation d'ensemble du liquide, dont le diamètre de rotation est entre 4/5 et 2/5 de celui du réacteur utilisé.

La vitesse de refroidissement est réglée avec une rampe de température qui peut aller de 100 à 3°C par heure.

L'obtention d'une forme cristalline particulière, plutôt que d'un mélange en proportions fixes de deux formes dépend des deux paramètres ci-dessus à la fois, dans un solvant déterminé, étant entendu que la vitesse d'agitation varie généralement en relation directe avec la vitesse de refroidissement.

Dans l'étape (c), le produit ainsi cristallisé est isolé selon les techniques conventionnelles et, il est micronisé.

L'isolement du produit peut prévoir par exemple le séchage du composé obtenu ; il a été démontré que l'étape de séchage, qu'elle soit effectuée en étuve ou dans un sécheur agité, ne modifie pas les structures cristallines obtenues en fin de cristallisation.

En choisissant les conditions appropriées pour les étapes (a) et (b), dans l'étape (c) on peut isoler quatre espèces différentes de SR 57746 A, à savoir la Forme I, la Forme II, la Forme III ou le mélange Forme I/III, dont les caractéristiques essentielles sont déterminables par analyse calorimétrique différentielle ("DSC", de l'anglais Differential Scanning Calorimetry) donnant, par des thermogrammes obtenus avec un calorimètre PERKIN-ELMER dans des conditions bien définies,

- la température de transition solide-solide; et
- l'enthalpie liée à cette transition.

L'analyse calorimétrique différentielle a été réalisée en utilisant un appareil DSC7 PERKIN-ELMER dont l'étalonnage a été effectué par rapport aux endothermes de fusion de l'indium ou du plomb et du cyclohexane. Pour cette analyse, on a utilisé de 3 à 6 mg de produit dans une coupelle d'aluminium sertie et percée sur le couvercle, dans une zone de température de 50 à 180°C, à une vitesse de chauffage de 10°C/minute, en utilisant l'azote en tant que gaz de balayage.

La température de transition solide-solide et l'enthalpie de transition constituent des caractéristiques essentielles en soi suffisantes à identifier chaque forme cristalline ou les mélanges de deux desdites formes.

Lesdites formes peuvent être également caractérisées par diffractométrie des rayons X de la poudre. Le profil de diffraction des rayons X de la poudre (angles de diffraction 2 θ de Bragg) a été établi avec un diffractomètre SIEMENS 500 TT avec un générateur 40 kV, monochromateur arrière, source Cu k x l, portoir en silicium et dans un domaine de balayage de 4° à 40° à raison de 1° par minute.

Selon une méthode avantageuse, l'étape (a) est effectuée en chauffant au reflux le SR 57746 A dans un mélange éthanol/acide chlorhydrique dans un rapport compris entre 95/5 et 70/30 jusqu'à dissolution complète et l'étape (b) est effectuée en refroidissant la solution ainsi obtenue avec une rampe de température de 3 à 100°C par heure jusqu'à 4°C environ, sans agitation. Selon ce mode opératoire avantageux. dans l'étape (c) on isole une forme cristalline du SR 57746 A, ci-après désignée "Forme I", caractérisée en ce qu'elle présente

- une température de transition solide-solide de 148,4 ± 1,6 °C
- une enthalpie de transition de 26,4 ± 1,1 J/g.

La Forme I du SR 57746 A ayant les caractéristiques ci-dessus constitue un aspect ultérieur de la présente invention.

Cette nouvelle forme cristalline a été aussi analysée par diffraction des rayons X de la poudre. L'étude qualitative des diffractogrammes a permis d'établir que la Forme I présente des raies caractéristiques (2 θ) à

- 9,9 ± 0,3 °
- 14,8 ± 0,3 °
- 20,8 ± 0,3 ° (intensité 100).

La Forme I est également obtenue lorsque, dans l'étape (b), on refroidit la solution en la laissant 8-15 heures à 0-5°C, toujours sans agitation.

Selon un autre mode opératoire avantageux, l'étape (a) est effectuée en chauffant au reflux dans l'éthanol absolu ou dans un mélange acétate d'éthyle/eau de 95/5 à 75/15 jusqu'à dissolution complète, le SR 57746 A étant présent dans cette solution à une concentration de 10-80 g/l, de préférence de 70 g/l dans le mélange acétate d'éthyle/eau ou de 5-150 g/l dans l'éthanol absolu.

L'étape (b), selon ce mode opératoire avantageux, est effectuée en refroidissant de la température de reflux à environ 5°C avec une rampe de température de 100 à 30°C par heure et une vitesse d'agitation de 100 à 600 tr/minute.

Dans l'étape (c), on isole ainsi une autre forme cristalline, ci-après désignée "Forme II", caractérisée en ce qu'elle présente

- · une température de transition solide-solide de 153,9 ± 1,1 °C
- · une enthalpie de transition de 24,1 ± 1,0 J/g.

La Forme II du SR 57746 A ayant les caractéristiques ci-dessus constitue un autre aspect de la présente invention.

Cette nouvelle forme cristalline a été également analysée par diffraction des rayons X de la poudre. L'étude qualitative des diffractogrammes a permis d'établir que la Forme II présente des raies caractéristiques ( 2 θ) à

- · 14,5 ± 0,3 ° (intensité 100).
- · 19,3 ± 0,3 °
- · 20,4 ± 0,3 °.

Selon un autre mode opératoire avantageux, l'étape (a) est effectuée en chauffant au reflux le SR 57746 A dans du diméthylsulfoxyde jusqu'à dissolution complète et

Le fait de pouvoir disposer de formes bien définies du SR 57746 A ou d'un mélange fixe Forme I/Forme III permet de préparer des compositions pharmaceutiques ayant une composition constante et reproductible.

De plus, l'obtention d'un produit ayant une granulométrie fine, par exemple par micronisation, permet - à activité constante - de diminuer sensiblement les doses efficaces pour obtenir le même résultat thérapeutique.

Plus particulièrement, il a été démontré que la forme microcristalline non seulement permet de diminuer la quantité de dosage présente dans les compositions pharmaceutiques mais, surtout, permet de rendre uniforme l'absorption par voie orale et d'avoir ainsi une réponse thérapeutique constante chez chaque patient que le produit soit administré à jeun ou avec le repas.

Une étude concernant la détermination de l'absorption in vitro du SR 57746 A - mélange Forme I/III micronisé a été effectuée en utilisant le modèle mono-couche CACO-2. Ce test, largement utilisé comme modèle épithélial intestinal prédictif pour l'absorption des médicaments (P. Artusson, Crit. Rev. Ther. Drug, 1991,8:305-330) a permis de mettre en évidence des différences significatives de dissolution et de perméabilité du SR 57746 A - mélange Forme I/III micronisé par rapport au SR 57746 A obtenu selon EP 0 101 381.

Les résultats montrent que, dans le milieu utilisé (solution de Hank additionnée de sérum foetal de veau à 10% et d'acide taurocholique), les vitesses de dissolution et de perméabilité sont significativement différentes pour le SR 57746 A - mélange Forme I/III micronisé par rapport au SR 57746 A obtenu selon EP 0 101 381. Plus particulièrement, il a été démontré que la dissolution et la perméabilité sont normalisées - à savoir rendues uniformes - après micronisation.

Ainsi, selon un autre de ses aspects, la présente invention a pour objet une composition pharmaceutique contenant, en tant que principe actif, le chlorhydrate de 1-[2-(2-naphtyl)éthyl]-4-(3-trifluorométhylphényl)-1,2,3,6-tétrahydropyridine sous une forme cristalline micronisée choisie parmi la Forme I, la Forme II, la Forme III telles que définies ci-dessus, et les mélanges Forme I/Forme III dans des rapports de 80/20 à 60/40, avantageusement de 70/30 à 65/35, de préférence d'environ 66/34.

L'administration des formes cristallines de l'invention peut être convenablement effectuée par voie orale, parentérale, sublinguale ou transdermique. La quantité de principe actif à administrer dépend de la nature et de la gravité des affections à traiter ainsi que du poids des malades. Néanmoins, la quantité de principe actif présent dans l'unité de dosage peut aller jusqu'à 10 mg (calculés en base libre) pour le produit non micronisé et peut être de 0.1 à 5 mg, avantageusement de 0,5 à 3 mg, de préférence 2 l'étape (b) est effectuée en refroidissant la solution ainsi obtenue avec une rampe de température de 3 à 100°C par heure et une vitesse d'agitation de 0 à 600 tr/minute.

Dans l'étape (c), on isole ainsi une autre forme cristalline, ci-après désignée "Forme III", caractérisée en ce qu'elle présente

- · une température de transition solide-solide de 141 ± 2 °C
- · une enthalpie de transition de 17,6 ± 0,5 J/g.

La Forme III du SR 57746 A ayant les caractéristiques ci-dessus constitue un autre aspect de la présente invention.

Selon un mode opératoire particulièrement avantageux, l'étape (a) est effectuée en chauffant le SR 57746 A dans un mélange éthanol/eau de 95/5 à 70/30, de préférence de 90/10 à 85/15, jusqu'à dissolution complète et l'étape (b) est effectuée en refroidissant avec une rampe de température de 5 à 30°C par heure, avantageusement jusqu'à 5°C avec une rampe de température de 10 à 20°C par heure, de préférence de 10°C par heure et à une vitesse d'agitation de 0 à 600 tr/minute, avantageusement de 200 à 400 tr/minute, de préférence de 400 tr/minute.

De façon inattendue, il a été constaté que, dans l'étape (c), on isole, de façon reproductible, un mélange Forme I/Forme III dans des rapports pondéraux de 80/20 à 60/40, avantageusement de 70/30 à 65/35, de préférence d'environ 66/34, comme il a été démontré par analyse calorimétrique différentielle.

Ce mélange est formé par des particules ayant un diamètre inférieur à 150 micromètres.

Les Formes I, II et III du SR 57746 A ainsi que le mélange des Formes I et III peuvent être micronisés dé façon à obtenir un principe actif pharmaceutique ayant une granulométrie inférieure à 50 micromètres, avantageusement inférieure à 30 micromètres, de préférence, pour au moins 80% des particules, inférieure à 10 micromètres.

La micronisation peut être effectuée dans un appareil classique permettant d'obtenir des microcristaux ayant une taille inférieure à 50 micromètres, par exemple dans un microniseur ALPINE 200 AS, en introduisant le SR 57746 A dans la chambre de micronisation (diamètre de 200 mm) à la vitesse de 15 à 50 kg/heure et à une pression de travail de 1 à 6,5 bar et en récuperant le produit dans une manche filtrante.

Les formes cristallines I, II, III du SR 57746 A, ainsi que les mélanges des Formes I et III dans des rapports de 80/20 à 60/40, avantageusement de 70/30 à 65/35, de préférence d'environ 66/34, micronisés, constituent un aspect particulièrement avantageux de la présente invention. mg (calculés en base libre) pour le produit micronisé. Les doses unitaires préférées comprendront généralement 0,5, 1, 1,5, 2, 2,5 ou 3 mg (calculés en base libre) de produit micronisé.

Ces doses unitaires seront administrées normalement une ou plusieurs fois par jour, par exemple une ou deux fois par jour, la dose globale chez l'homme étant variable entre 0,5 et 20 mg par jour, avantageusement entre 1 et 10 mg par jour (calculés en base libre) pour le produit non micronisé et de 0,2 à 10 mg par jour, avantageusement entre 1 et 6 mg par jour (calculés en base libre), pour le produit micronisé.

Dans les formes unitaires des compositions pharmaceutiques de la présente invention, le principe actif est administré, de préférence en mélange avec des supports pharmaceutiques classiques, aux animaux et aux êtres humains pour le traitement des maladies indiquées notamment dans les brevets US 5 026 716, US 5 109 005, US 5 270 320, US 5 292 745 et US 5 378 709, en particulier pour le traitement de la neurodégénérescence, notamment la sclérose latérale amyotrophique. Les formes unitaires d'administration appropriées comprennent de préférence les formes par voie orale telles que les comprimés éventuellement sécables, les gélules, les poudres, les granules, les formes d'administration sublinguale et buccale, les formes d'administration transdermique pouvant également être préparées en utilisant les nouvelles formes cristallines.

Lorsqu'on prépare une composition solide sous forme de comprimés, on mélange le principe actif avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Le principe actif peut être formulé également sous forme de microcapsules, éventuellement avec un ou plusieurs supports ou additifs.

Dans les compositions pharmaceutiques selon la présente invention, le principe actif peut être aussi sous forme de complexe d'inclusion dans des cyclodextrines, leurs éthers ou leurs esters.

Les exemples suivants illustrent l'invention.

EXEMPLE 1

Un mélange de 19,5 g de chlorhydrate de 1-[2-(2-naphtyl)éthyl]-4-(3-trifluorométhylphényl)-1,2,3,6-tétrahydropyridine brut, 95 ml d'éthanol absolu et 4,65 ml d'acide chlorhydrique à 37% est chauffé au reflux sous agitation jusqu'à dissolution complète, puis on laisse refroidir, toujours sous agitation. Lorsque les premiers cristaux commencent à se former (vers 63°C), l'agitation est arrêtée et le mélange réactionnel est maintenu à 0-5°C pendant une nuit. Après filtration, le produit est réempâté deux fois dans 30 ml d'éthanol absolu, puis seché pendant une nuit à 40°C sous vide.

Dans ces conditions, on a obtenu 12,8 g de Forme I du chlorhydrate de 1-[2-(2-naphtyl)éthyl]-4-(3-trifluorométhylphényl)-1,2,3,6-tétrahydropyridine (SR 57746 A - Forme I).

A l'analyse calorimétrique différentielle, le SR 57746 A - Forme I obtenu dans cette préparation a présenté

- une température de transition solide-solide de 148-149°C
- une enthalpie de transition de 26,4 J/g.

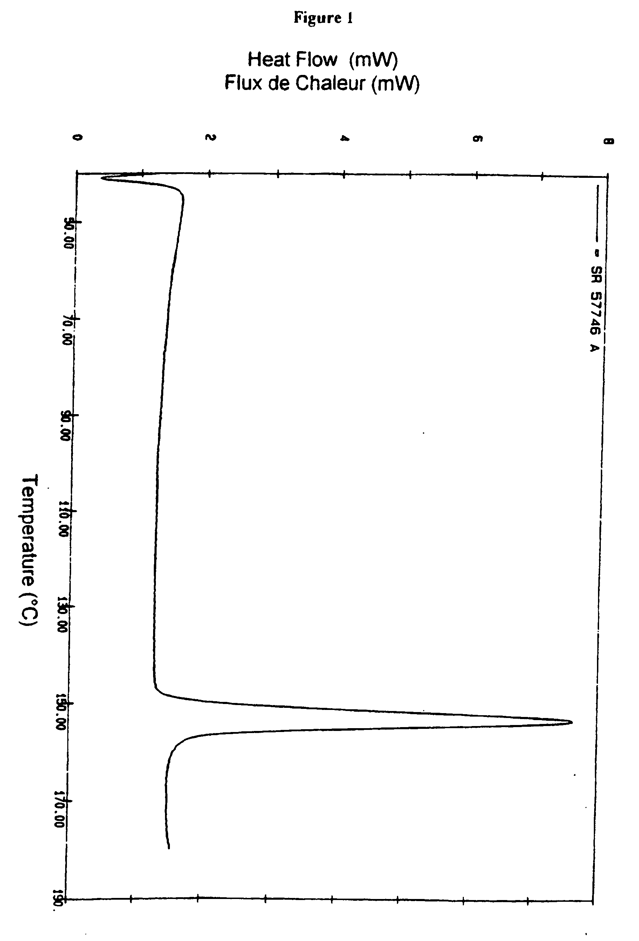

Le thermogramme relatif est consigné dans la Figure 1.

A l'analyse de diffraction des rayons X de la poudre, avec un diffractomètre SIEMENS 500 TT dans les conditions données ci-dessus, le SR 57746 A - Forme I obtenu dans cette préparation présente des raies caractéristiques (angles 2 θ de Bragg) à 9,8°; 14,7° et 20,7° (intensité relative: 100).

Le profil de diffraction des rayons X de la poudre (angles de diffraction) du SR 57746 A - Forme I de cette préparation est donné par les raies significatives rassemblées dans le Tableau 1, avec l'intensité relative, exprimée en pourcentage par rapport à la raie la plus intense.

TABLEAU 1

| SR 57746 A - Forme I | |
|---|---|
| Bandes de diffraction (angles de Bragg 2θ) | Intensité relative |
| 9,798 | 23,44 |
| 14,758 | 79.68 |
| 15,174 | 45.73 |
| 16,584 | 49,31 |
| 16,922 | 34,30 |
| 17,458 | 35,91 |
| 17,814 | 21,48 |
| 18,403 | 32,33 |
| 20,741 | 100,00 |
| 21,367 | 29,91 |
| 22,310 | 28,98 |
| 24,482 | 22,75 |
| 24.768 | 67,67 |
| 25,644 | 40,18 |
| 28,803 | 39,03 |

EXEMPLE 2

Dans un réacteur calorimétrique METTLER RC1 muni d'un agitateur impeller de 8 cm de diamètre, un mélange de 70 g de chlorhydrate de 1-[2-(2-naphtyl)éthyl]-4-(3-trifluorométhylphényl)-1,2,3,6-tétrahydropyridine brut et de 1 l d'éthanol absolu est chauffé au reflux jusqu'à dissolution complète du produit. La solution ainsi obtenue est refroidie avec une vitesse de refroidissement de 80°C par heure et une vitesse d'agitation de 500 tr/minute jusqu'à 10°C. Le précipité ainsi obtenu est filtré et seché une nuit à 45°C sous vide.

Dans ces conditions, on a obtenu la Forme II du chlorhydrate de 1-[2-(2-naphtyl)éthyl]-4-(3-trifluorométhylphényl)-1,2,3,6-tétrahydropyridine (SR 57746 A - Forme II).

A l'analyse calorimétrique différentielle, le SR 57746 A - Forme II obtenu dans cette préparation a présenté

· une température de transition solide-solide de 153-155°C
· une enthalpie de transition de 24,1 J/g.

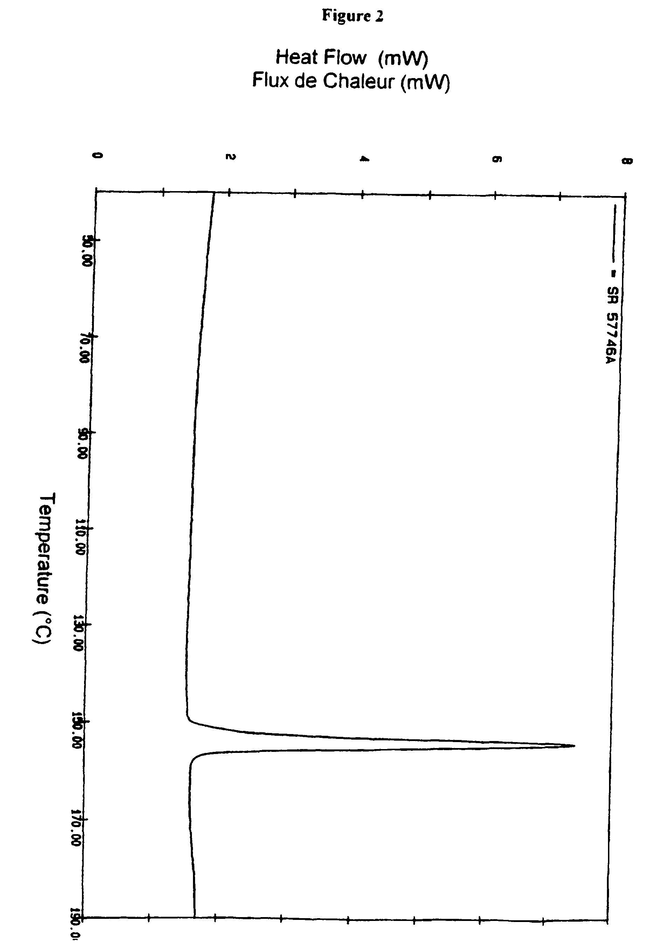

Le thermogramme relatif est consigné dans la Figure 2.

A l'analyse de diffraction des rayons X de la poudre, avec un diffractomètre SIEMENS 500 TT dans les conditions données ci-dessus, le SR 57746 A - Forme II obtenu dans cette préparation présente des raies caractéristiques (angles 2 θ de Bragg) à 14,3° (intensité relative: 100), 19,2° et 20,5°.

Le profil de diffraction des rayons X de la poudre (angles de diffraction) du SR 57746 A - Forme II de cette préparation est donné par les raies significatives rassemblées dans le Tableau 2, avec l'intensité relative, exprimée en pourcentage par rapport à la raie la plus intense.

TABLEAU 2

| SR 57746 A - Forme II | |
|---|---|
| Bandes de diffraction (angles de Bragg 2θ) | Intensité relative |
| 14,348 | 100,00 |

TABLEAU 2   (suite)

| SR 57746 A - Forme II | |
| --- | --- |
| Bandes de diffraction (angles de Bragg 2θ) | Intensité relative |
| 16,300 | 21,68 |
| 16,748 | 57,51 |
| 17,209 | 68,98 |
| 19,173 | 34,10 |
| 20,147 | 37,38 |
| 20.493 | 28,52 |
| 20,832 | 33,62 |
| 24,332 | 37,28 |
| 24,902 | 24,57 |
| 25,237 | 41,71 |
| 25,817 | 24.57 |

EXEMPLE 3

Un mélange de 2 g de chlorhydrate de 1-[2-(2-naphtyl)éthyl]-4-(3-trifluorométhylphényl)-1,2,3,6-tétrahydropyridine brut et de 50 ml de diméthylsulfoxyde est chauffé au reflux jusqu'à dissolution complète, on laisse refroidir le mélange pendant une nuit, puis on récupère le produit cristallin et on le sèche sous vide à 45° C pendant une nuit.
Dans ces conditions, un a obtenu la Forme III du chlorhydrate de 1-[2-(2-naphtyl)éthyl]-4-(3-trifluorométhylphényl)-1,2,3,6-tétrahydropyridine (SR 57746 A - Forme III).
A l'analyse calorimétrique différentielle, le SR 57746 A - Forme III obtenu dans cette préparation a présenté

- une température de transition solide-solide de 141-142°C
- une enthalpie de transition de 17,6 J/g.

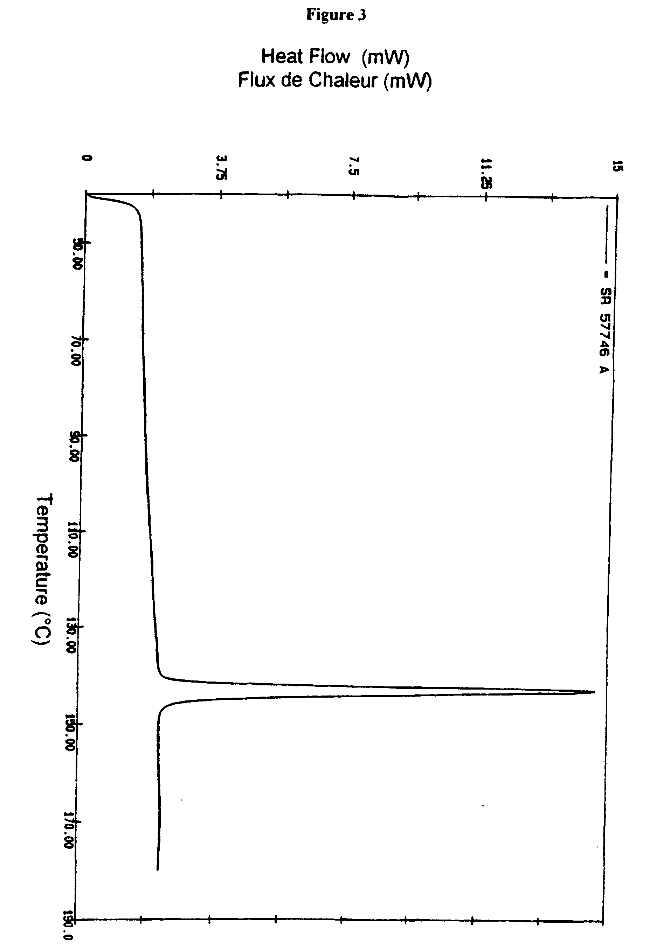

Le thermogramme relatif est consigné dans la Figure 3.

EXEMPLE 4

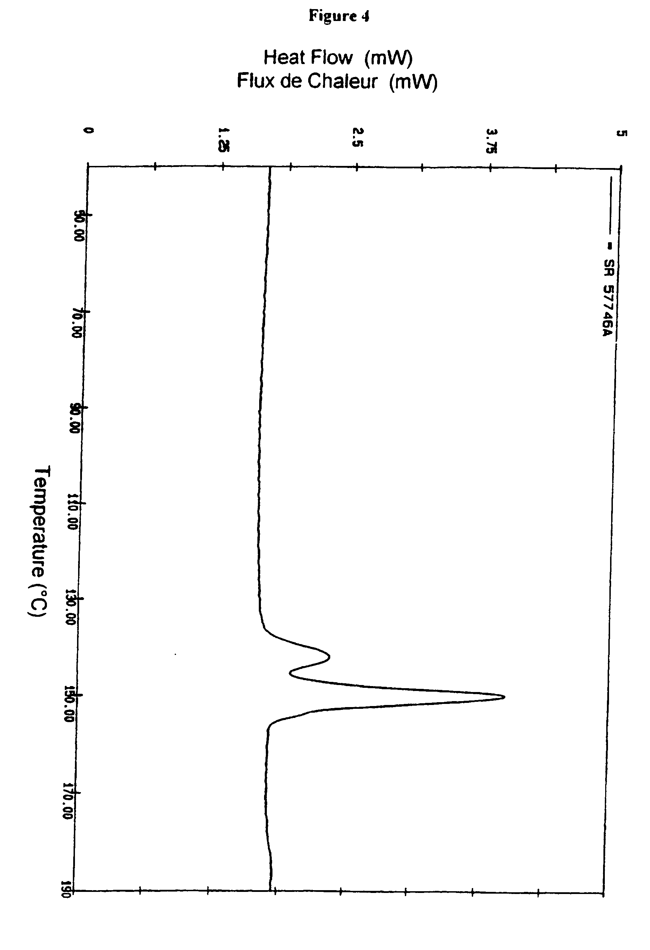

Un mélange de 100 g de chlorhydrate de 1-[2-(2-naphtyl)éthyl]-4-(3-trifluorométhylphényl)-1,2,3,6-tétrahydropyridine brut dans 1 l d'un mélange éthanol/eau 90/10 est chauffé au reflux sous agitation jusqu'à dissolution complète du produit. La solution ainsi obtenue est refroidie de la température de reflux à 5°C sous agitation impeller à 400 tr/minute à une vitesse de refroidissement de 10°C/heure. Le produit cristallin ainsi obtenu est filtré et seché à 45°C sous vide pendant une nuit.
Dans ces conditions, on a obtenu le chlorhydrate de 1-[2-(2-naphtyl)éthyl]-4-(3-trifluorométhylphényl)-1,2,3,6-tétrahydropyridine sous forme de mélange Forme I/Forme III, dans un rapport 65,7/34,3 (SR 57746 A - Forme I/III).
A l'analyse calorimétrique différentielle, le SR 57746 A - Forme I/III obtenu dans cette préparation présente un thermogramme, consigné dans la Figure 4, qui montre uniquement les deux pics caractéristiques correspondant aux Formes I et III.

EXEMPLES 5 et 6

En opérant comme décrit dans l'Exemple 2, dans deux préparations différentes on a varié la vitesse de refroidissement et la vitesse d'agitation de la façon suivante:

- refroidissement à 100°C/heure et agitation à 600 tr/minute (Ex. 5);
- refroidissement à 30°C/heure et agitation à 300 tr/minute (Ex. 6);

Dans ces conditions on a obtenu le SR 57746 A - Forme II.

Il a donc été constaté qu'en opérant dans l'éthanol absolu à une concentration de 70 g/l, l'obtention de la Forme II dépend de la vitesse de refroidissement et de la vitesse d'agitation selon une équation linéaire du type y = ax + b.

Pour l'obtention de la Forme II dans ces conditions, l'équation est la suivante

$$R_{max} = 4,23.V + 170,51$$

dans laquelle $R_{max}$ est la vitesse d'agitation en tr/minute et V est la vitesse de refroidissement en °C/heure. Par conséquent, pour l'obtention de la Forme II, pour une vitesse de refroidissement donnée, la vitesse d'agitation doit être inférieure ou égale à $R_{max}$.

EXEMPLE 7

Un mélange de 15 g de chlorhydrate de 1-[2-(2-naphtyl)éthyl]-4-(3-trifluorométhylphényl)-1,2,3,6-tétrahydropyridine brut dans 1 l d'un mélange acétate d'éthyle/eau 90/10 est chauffé au reflux jusqu'à dissolution complète du produit sous agitation avec un agitateur impeller de 8 cm de diamètre. La solution ainsi obtenue est refroidie à 60°C par heure avec une vitesse d'agitation de 150 tr/minute, jusqu'à 5°C, puis on filtre le précipité ainsi obtenu et on le sèche sous vide. On obtient ainsi le SR 57746 A - Forme II identique au produit obtenu selon l'Exemple 2.

EXEMPLES 8-11

Dans quatre préparations différentes, le chlorhydrate de 1-[2-(2-naphtyl)éthyl]-4-(3-trifluorométhylphényl)-1,2,3,6-tétrahydropyridine brut dans un mélange acétate d'éthyle/eau 92/8 à une concentration de 70g/l (volume réactionnel: 1,3 l) est chauffé au reflux dans un réacteur RC 1 couplé avec un moniteur de particules PARTEC® 100 de la société LASENTEC et muni d'un agitateur impeller de 8 cm de diamètre. Après dissolution complète, dans les quatre préparations la solution a été refroidie dans les conditions suivantes:

- · refroidissement à 100°C/heure et agitation à 400 tr/minute (Ex. 8);
- · refroidissement à 80°C/heure et agitation à 300 tr/minute (Ex. 9);
- · refroidissement à 50°C/heure et agitation à 200 tr/minute (Ex. 10);
- · refroidissement à 30°C/heure et agitation à 100 tr/minute (Ex. 11);

Dans ces conditions on a obtenu le SR 57746 A - Forme II.

Il a été constaté qu'en opérant dans un mélange acétate d'éthyle/eau 92/8 à une concentration de 70g/l, l'obtention de la Forme II dépend de la vitesse de refroidissement et de la vitesse d'agitation selon l'équation linéaire suivante

$$R_{max} = 4,14. V - 18,9$$

dans laquelle $R_{max}$ est la vitesse d'agitation en tr/minute et V est la vitesse de refroidissement en °C/heure.

Pour l'obtention de la Forme II, il faut donc que, pour une vitesse de refroidissement donnée, la vitesse d'agitation soit inférieure ou égale à $R_{max}$.

EXEMPLE 12

On chauffe au reflux sous agitation jusqu'à dissolution complète le chlorhydrate de 1-[2-(2-naphtyl)éthyl]-4-(3-trifluorométhylphényl)-1,2,3,6-tétrahydropyridine brut dans un mélange acétone/eau 90/10 à une concentration de 60,6 g/l. En opérant ensuite comme décrit dans l'Exemple 4, on obtient le SR 57746 A - Forme I/III dans un rapport 80/20.

EXEMPLE 13

On chauffe au reflux sous agitation jusqu'à dissolution complète le chlorhydrate de 1-[2-(2-naphtyl)éthyl]-4-(3-trifluorométhylphényl)-1,2,3,6-tétrahydropyridine brut dans du méthanol à une concentration de 100 g/l. En opérant ensuite comme décrit dans l'Exemple 4, on obtient le SR 57746 A - Forme I/III dans un rapport 80/20 identique au produit de l'Exemple 12.

EXEMPLE 14

On chauffe au reflux sous agitation jusqu'à dissolution complète le chlorhydrate de 1-[2-(2-naphtyl)éthyl]-4-(3-trifluorométhylphényl)-1,2,3,6-tétrahydropyridine brut dans un mélange éthanol/eau 70/30 à une concentration de 100 g/l. En opérant ensuite comme décrit dans l'Exemple 4, on obtient le SR 57746 A - Forme I/III dans un rapport 65,7/34,3 identique au produit de l'Exemple 4.

EXEMPLE 15

On introduit dans la chambre de micronisation (diamètre 200 mm) d'un microniseur ALPINE 200 AS 24 kg de SR 57746 A - Forme I/III, décrit dans l'Exemple 4, à une vitesse de 25 kg/heure et à une pression de travail de 6,5 bar et on recupère le produit ainsi micronisé dans une manche filtrante. On obtient ainsi le SR 57746 A - Forme I/III ayant une distribution particulaire selon laquelle la totalité des particules a une taille inférieure à 20 micromètres et 85% des particules ont une taille inférieure à 10 micromètres.
L'analyse calorimétrique différentielle du produit micronisé ainsi obtenu montre que les températures de transition ne sont pas affectées par la micronisation. Lesdites transitions sont du type solide-solide. Le SR 57746 A se dégrade avant la fusion, qui commence à 250 °C.

EXEMPLE 16

Composition pharmaceutique contenant, en tant que principe actif, le SR 57746 A

- Forme I/III (micronisé) selon l'Exemple 15 ci-dessus:

| Principe actif | 2,192 mg |
|---|---|
| Amidon de maïs | 141,218 mg |
| Silice colloïdale anhydre | 0,200 mg |
| Stéarate de magnésium | 0,400 mg |

Le principe actif est tamisé à 0,2 mm, puis prémélangé avec les excipients. Ce mélange est tamisé à 0,315 mm, remélangé, puis à nouveau tamisé à 0,315 mm. Après un dernier mélange, on introduit la composition dans des gélules de gélatine n° 3, à raison de 170 mg de composition contenant une quantité de SR 57746 A - Forme I/III correspondant à 2 mg de 1-[2-(2-naphtyl)éthyl]-4-(3-trifluorométhylphényl)-1,2,3,6-tétrahydropyridine base.

**Revendications**

1. Procédé pour la cristallisation du chlorhydrate de 1-[2-(2-naphtyl)éthyl]-4-(3-trifluorométhylphényl)-1,2,3,6-tétrahydropyridine (SR 57746 A) **caractérisé en ce que** :

   (a) on chauffe au reflux le SR 57746 A dans un mélange éthanol/eau de 95/5 à 70/30 jusqu'à dissolution complète ;
   (b) on refroidit la solution ainsi obtenue avec une rampe de température de 5 à 30°C par heure et une vitesse d'agitation de 0 à 600 tr/minute ; et
   (c) on isole le mélange Forme I/Forme III du chlorhydrate de 1-[2-(2-naphtyl)éthyl]-4-(3-trifluorométhylphényl)-1,2,3,6-tétrahydropyridine dans un rapport de 80/20 à 60/40 et on le micronise.

2. Procédé selon la revendication 1, **caractérisé en ce que** dans l'étape (a) on utilise un mélange éthanol/eau 90/10, l'étape (b) est effectuée en refroidissant la solution ainsi obtenue jusqu'à 5°C avec une rampe de température de 10 à 20°C par heure et une vitesse d'agitation de 200 à 400 tr/minute et dans l'étape (c), on isole le mélange Forme I/Forme III du chlorhydrate de 1-[2-(2-naphtyl)éthyl]-4-(3-trifluorométhylphényl)-1,2,3,6-tétrahydropyridine dans un rapport de 70/30 à 65/35.

3. Procédé selon la revendication 2, **caractérisé en ce que** dans l'étape (b) la rampe de température est de 10 °C par heure et la vitesse d'agitation de 400 tr/minute et, dans l'étape (c), on isole le mélange Forme I/Forme III du chlorhydrate de 1-[2-(2-naphtyl)éthyl]-4-(3-trifluorométhylphényl)-1,2,3,6-tétrahydropyridine dans un rapport d'environ 66/34.

4. Procédé selon l'une des revendications 1 à 3, **caractérisé en ce qu'**on utilise un agitateur mobile à palette.

5. Mélange de Forme I et de Forme III du chlorhydrate de 1-[2-(2-naphtyl)éthyl]-4-(3-trifluorométhylphényl)-1,2,3,6-tétrahydropyridine, dans un rapport Forme I/Forme III de 80/20 à 60/40, ledit mélange étant micronisé et présentant une granulométrie inférieure à 50 µm,
ladite Forme I présentant

* une température de transition solide-solide de 148,4 ± 1,6°C
* une enthalpie de transition de 26,4 ± 1,1 J/g

ladite Forme III présentant

* une température de transition solide-solide de 141 ± 2° C
* une enthalpie de transition de 17,6 ± 0,5 J/g.

6. Mélange selon la revendication 5, **caractérisé en ce que** le diffractogramme des rayons X de la poudre de la Forme I présente des raies caractéristiques (angles 2θ de Bragg) à

* 9,9 ± 0,3°
* 14,8 ± 0,3°
* 20,8 ± 0,3° (intensité 100).

7. Mélange selon la revendication 5 ou 6, présentant une granulométrie inférieure à 30 µm

8. Mélange selon la revendication 7, dans lequel au moins 80% des particules ont une granulométrie inférieure à 10 µm.

9. Mélange selon l'une des revendication 5 à 8, dans un rapport Forme I/Forme III de 70/30 à 65/35.

10. Mélange selon la revendication 9, dans un rapport Forme I/Forme III d'environ 66/34.

11. Composition pharmaceutique contenant, en tant que principe actif, un mélange de Forme I et de Forme III de chlorhydrate de 1-[2-(2-naphtyl)éthyl]-4-(3-trifluorométhylphényl)-1,2,3,6-tétrahydropyridine tel que défini dans l'une des revendications 5 à 10.

12. Composition selon la revendication 11, **caractérisée en ce que** chaque unité de dosage contient une quantité de principe actif correspondant à une dose choisie parmi 0,5, 1, 1,5, 2, 2,5 ou 3 mg de base libre.


**Patentansprüche**

1. Verfahren zur Kristallisation von 1-[2-(2-Naphthyl)-ethyl]-4-(3-trifluormethylphenyl)-1,2,3,6-tetrahydropyridin-Hydrochlorid (SR 57746 A), **dadurch gekennzeichnet, dass**:

(a) das SR 57746 A in einem Ethanol/Wasser-Gemisch von 95/5 bis 70/30 bis zum vollständigen Lösen auf Rückflusstemperatur erwärmt wird;
(b) die auf diese Weise erhaltene Lösung mit einer Temperaturrampe von 5 bis 30 °C pro Stunde und einer Rührgeschwindigkeit von 0 bis 600 U/min abgekühlt wird; und
(c) das Form I/Form III-Gemisch von 1-[2-(2-Naphthyl)-ethyl]-4-(3-trifluormethylphenyl)-1,2,3,6-tetrahydropyridin-Hydrochlorid in einem Verhältnis von 80/20 bis 60/40 abgetrennt und mikronisiert wird.

2. Verfahren nach Anspruch 1, **dadurch gekennzeichnet, dass** in Schritt (a) ein Ethanol/Wasser-Gemisch 90/10 verwendet wird, Schritt (b) durchgeführt wird, indem die so erhaltene Lösung mit einer Temperaturrampe von 10 bis 20 °C pro Stunde und einer Rührgeschwindigkeit von 200 bis 400 U/min auf 5 °C abgekühlt wird und in Schritt (c) das Gemisch Form I/Form III von 1-[2-(2-Naphthyl)-ethyl]-4-(3-trifluormethylphenyl)-1,2,3,6-tetrahydropyridin-Hydrochlorid in einem Verhältnis 70/30 bis 65/35 abgetrennt wird.

3. Verfahren nach Anspruch 2, **dadurch gekennzeichnet, dass** in Schritt (b) die Temperaturrampe 10 °C pro Stunde beträgt und die Rührgeschwindigkeit 400 U/min ist und in Schritt (c) das Gemisch Form I/Form III von 1-[2-(2-Naphthyl)-ethyl]-4-(3-trifluormethylphenyl)-1,2,3,6-tetrahydropyridin-Hydrochlorid in einem Verhältnis von etwa 66/34 abgetrennt wird.

4. Verfahren nach einem der Ansprüche 1 bis 3, **dadurch gekennzeichnet, dass** ein mobiles Propeller-Rührwerk verwendet wird.

5. Gemisch von Form I und Form III von 1-[2-(2-Naphthyl)-ethyl]-4-(3-trifluormethylphenyl)-1,2,3,6-tetrahydropyridin-Hydrochlorid in einem Verhältnis Form I/Form III von 80/20 bis 60/40, wobei das Gemisch mikronisiert ist und eine Korngröße unter 50 μm

aufweist, wobei Form I aufweist:

* eine Übergangstemperatur fest-fest von 148,4 ± 1,6 °C
* eine Übergangsenthalpie von 26,4 ± 1,1 J/g

und wobei Form III aufweist:

* eine Übergangstemperatur fest-fest von 141 ± 2 °C
* eine Übergangsenthalpie von 17,6 ± 0,5 J/g.

6. Gemisch nach Anspruch 5, **dadurch gekennzeichnet, dass** das Röntgendiffraktogramm des Pulvers der Form I charakteristische Reflexe (Bragg-Winkel 2θ) von

* 9,9 ± 0,3°
* 14,8 ± 0,3°
* 20,8 ± 0,3° (Intensität 100)

aufweist.

7. Gemisch nach Anspruch 5 oder 6, das eine Korngröße unter 30 μm aufweist.

8. Gemisch nach Anspruch 7, wobei mindestens 80 % der Partikel eine Korngröße unter 10 μm aufweisen.

9. Gemisch nach einem der Ansprüche 5 bis 8 in einem Verhältnis Form I/Form III von 70/30 bis 65/35.

10. Gemisch nach Anspruch 9 in einem Verhältnis Form I/Form III von etwa 66/34.

11. Pharmazeutische Zusammensetzung, die als Wirkstoff ein Gemisch der Form I und der Form III von 1-[2-(2-Naphthyl)-ethyl]-4-(3-trifluormethylphenyl)-1,2,3,6-tetrahydropyridin-Hydrochlorid nach einem der Ansprüche 5 bis 10 enthält.

12. Zusammensetzung nach Anspruch 11, **dadurch gekennzeichnet, dass** jede Dosiereinheit eine Wirkstoffmenge enthält, die einer Dosis entspricht, die unter 0,5, 1, 1,5, 2, 2,5 oder 3 mg der freien Base ausgewählt ist.

**Claims**

1. Method of crystallizing 1-[2-(naphth-2-yl)ethyl]-4-(3-trifluoromethylphenyl)-1,2,3,6-tetrahydropyridine hydrochloride (SR 57746 A), **characterized in that**:

    (a) the SR 57746 A is heated by refluxing in a 95/5 to 70/30 ethanol/water mixture until dissolution is complete,
    (b) the resulting solution is cooled with a temperature gradient of 5 to 30°C per hour and a stirrer speed of 0 to 600 rpm, and
    (c) a form I/ form III mixture of 1-[2-(naphth-2-yl)ethyl]-4-(3-trifluoromethylphenyl)-1,2,3,6-tetrahydropyridine hydrochloride in a ratio of 80/20 to 60/40 is isolated.

2. Method according to claim 1, **characterized in that** a 90/10 ethanol/water mixture is used in step (a), step (b) is carried out by cooling the resulting solution to 5°C with a temperature gradient of 10 to 20°C per hour and a stirrer speed of 200 to 400 rpm, and a form I/ form III mixture of 1-[2-(naphth-2-yl)ethyl]-4-(3-trifluoromethylphenyl)-1,2,3,6-tetrahydropyridine hydrochloride in a ratio of 70/30 to 65/35 is isolated in step (c).

3. Method according to claim 2, **characterized in that** the temperature gradient is 10°C per hour and the stirrer speed is 400 rpm in step (b), and a form I/ form III mixture of 1-[2-(naphth-2-yl)ethyl]-4-(3-trifluoromethylphenyl)-1,2,3,6-tetrahydropyridine hydrochloride in a ratio of about 66/34 is isolated in step (c).

4. Method according to one of claims 1 to 3, **characterized in that** a paddle stirrer is used.

5. A mixture of form I and form III of 1-[2-(naphth-2-yl)ethyl]-4-(3-trifluoromethylphenyl)-1,2,3,6-tetrahydropyridine hydrochloride in a form I/form III ratio of 80/20 to 60/40, said form I having :

   * a solid-solid transition temperature of 148.4 ± 1.6°C; and
   * an enthalpy of transition of 26.4 ± 1.1 J/g ;

   said form III having:

   * a solid-solid transition temperature of 141 ± 2°C; and
   * an enthalpy of transition of 17.6 ± 0.5 J/g.

6. Mixture according to claim 5, **characterized in that** X-ray powder diffraction pattern has characteristic lines (Bragg angles 2 θ) at:

   * 9.9 ± 0.3°
   * 14.8 ± 0.3°
   * 20.8 ± 0.3° (intensity: 100).

7. Mixture according to claim 5 or 6, having a particle size below 30 micrometers.

8. Mixture according to claim 7, in which at least 80% of the particles have a particle size below 10 micrometers.

9. Mixture according to anyone of claims 5 to 8, in a form I/form III ratio of 70/30 to 65/35.

10. Mixture according to claim 9, in a form I/form III ratio of about 66/34.

11. Phatmaceutical composition containing, as active principle a form I/form III mixture of 1-[2-(naphth-2-yl)ethyl]-4-(3-trifluoromethylphenyl)-1,2,3,6-tetrahydro pyridine hydrochloride as defined in anyone of claims 5 to 10.

12. Composition according to claim 11, **characterized in that** each dosage unit contains an amount of micronized active principle which corresponds to a dose selected from 0.5, 1, 1.5, 2, 2.5 and 3 mg of free base.

Figure 1

Heat Flow (mW)
Flux de Chaleur (mW)

## Figure 2

Heat Flow (mW)
Flux de Chaleur (mW)

## Figure 3

Heat Flow  (mW)
Flux de Chaleur (mW)

Figure 4

EP 0 950 050 B1

Heat Flow (mW)
Flux de Chaleur (mW)

Temperature (°C)

= SR 57746A